# EUROPEAN PATENT SPECIFICATION

(11) **EP 4 531 700 B1**
(45) Date of publication and mention of the grant of the patent: **30.09.2026**
(21) Application number: 23729080.4
(22) Date of filing: 25.05.2023
(51) Int. Cl.: A61B 10/00, G01N 1/20

(54) **LIQUID SAMPLER FOR FAST CAPTURE OF INITIAL VOLUME OF A LIQUID FLOW**
FLÜSSIGKEITSPROBENNEHMER ZUR SCHNELLEN ERFASSUNG DES AUSGANGSVOLUMENS EINES FLÜSSIGKEITSSTROMS
ÉCHANTILLONNEUR DE LIQUIDE POUR LA CAPTURE RAPIDE D'UN VOLUME INITIAL D'UN ÉCOULEMENT DE LIQUIDE

(30) Priority: 25.05.2022 US 202263345737 P
(43) Date of publication of application: 09.04.2025
(73) Proprietor: DNA Genotek Inc., Kanata, Ontario K2V 1C2 (CA)
(72) Inventor: BEYERS, Koen Catharina Lodewijk, 2990 Wuustwezel (BE); RÍOS CORTÉS, Alejandra, 3070 Kortenberg (BE); VANKERCKHOVEN, Vanessa Vicky Jill, 2610 Wilrijk (BE)
(74) Representative: Winger
(86) International application number: PCT/EP2023/064129
(87) International publication number: WO 2023/227748

(56) References cited:
- WO-A1-2014/037152
- WO-A2-2021/069454
- KR-B1- 101 876 699

## Description

### Field of the invention

The present invention relates to field of liquid collection devices, in particular to liquid sampling devices that capture a predetermined volume of an initial portion of a liquid flow, such as first-void urine samples for diagnostic purposes.

### Background of the invention

With the development of highly sensitive nucleic acid amplification testing of pathogenic nucleic acids, self-collected first-void urine has become a valuable non-invasive sample for diagnostic purposes, for instance for the detection of urogenital infections such as Chlamydia trachomatis, as well as other sexually transmitted infections. Results of this testing method, however, are only conclusive if the sampled urine fraction is not diluted or contaminated by the subsequent mid-stream urine. Moreover, even within the first-void volume of urine, there are variations of the microorganism load in urine samples which depend on the precise initial volume sampled, e.g. only a small fraction of the first-void urine.

Sampling only small volume fractions of urine can be challenging in practice, especially if the collection of urine is into a small-sized container, urine flow is fast, or if the urine stream has to be interrupted for correct sampling. This often causes discomfort to the user and frequently leads to unhygienic conditions while the urine sample is taken. The average donor urinates at about 16 mL/sec, making it difficult to collect small initial volumes - for instance, the initial 1-3 mL of first-void urine would be collected within a very short time period (milliseconds) once urination begins.

Therefore, a need for liquid sampling device exists which allow the precise sampling of an initial volume of a liquid flow, e.g. urine, and which are hygienic and comfortable in their use.

WO2014/037152 relates to a liquid sampling device for capturing a first portion of a liquid flow. The device comprises an inlet, an outlet, and a guide with a displaceable element which, in a first position, is capturing a first portion of the liquid flow, e.g. the first-void urine, into a reservoir, and which, in a second position, is blocking the access to the reservoir and is passing subsequent liquid to the outlet instead. The displaceable element moves in transverse direction to the liquid flow and has lifting means. Although being suitable for the sampling of first-void urine, it remains challenging for this device to restrict the quantity of sampled first-void urine to a small initial volume fraction (e.g. 1-3 mL) thereof.

WO2021/069454 discloses a liquid sampling device for small initial volumes, in which a closure member is displaced relative to a valve casing while an initial volume of a liquid flow is being sampled through a sample outlet. The displacement of the closure member is driven by the buoyancy force exerted on a lifting member of the liquid sampling device. A gate of the closure member is being lifted during collection of the initial volume, thereby establishing a fluid pathway for the subsequent volume of the liquid flow between an inlet and an outlet conduit. At the same time, a stem of the closure member is obstructing any further liquid flow through the sample outlet. The surface of the gate that is facing the inlet conduit is grooved. The groove extends from the gate downwards to the stem and redirects the liquid flow from the inlet conduit towards and through the sample outlet during collection of the initial volume of the flow.

There is a need for liquid sampling devices that allow for capturing small initial volumes of urine under hygienic conditions and that are further designed for fast capture of initial volumes of a liquid flow. A fast capture is desirable for the user on the one hand, and for higher-quality first-void urine samples on the other hand. In particular, collecting very small volume first-void urine samples, e.g. less than about 3 mL, proves to be challenging with the available liquid sampling devices, due to their limited capture speed.

It is therefore desirable to further improve existing liquid sampling devices, so that a faster capture of the initial volume of the liquid flow to be sampled can be achieved.

### Summary of the invention

It is an object of embodiments of the present invention to capture initial volumes of liquid flow at a higher speed. This objective is accomplished by a liquid sampling device and kit of parts according to the present invention.

The present invention relates to a device for fast sampling of an initial volume of a liquid flow. The device comprises a casing with a fluid inlet for receiving the liquid flow, a sample outlet for draining the initial volume of the liquid flow, and a fluid outlet for draining a subsequent volume of the liquid flow. A passageway extends inside the casing between the fluid inlet and the fluid outlet and provides fluid communication between the fluid inlet and the fluid outlet and further between the fluid inlet and the sample outlet. The device also comprises an elongated closure member that is slidably supported in the casing. The closure member has a head portion and a stem, and a lifting member connected to or formed in the stem. The lifting member is a float adapted for moving the closure member from a sampling position to a diverting position while the initial volume of the liquid flow is being collected into a receptacle, connectable to the sample outlet of the casing. The head portion is configured to obstruct the liquid flow through the fluid outlet when the closure member is slid into the sampling position, thus preventing the initial volume of the liquid flow from being transferred to the fluid outlet. A lower portion of the stem, distal to the head portion, is configured to obstruct the liquid flow through the sample outlet when the closure member is slid into the diverting position, thus ensuring that the subsequent volume of the liquid flow is transferred to the fluid outlet. The head portion is positioned in the passageway when the closure member is slid to the sampling position and the lower portion of the stem is extending through and at least partially into said passageway when the closure member is slid into the diverting position. Furthermore, an upper portion of the stem, proximate to the head portion, comprises at least one longitudinal slit, which stretches across the sample outlet to fluidly connect a front side of the stem facing the fluid inlet in the casing to an opposing rear side of the stem when the closure member is slid into the sampling position, thus allowing the initial volume of the liquid flow to exit said sample outlet via the at least one slit.

The one or more slits in the upper stem portion of the closure member provide the stem with a slotted, open central structure that fluidly connects a front side of the stem, facing the fluid inlet in the casing, to a rear side of the stem, which is facing the fluid outlet in the casing. This open structure allows for a significant reduction in the weight of the closure member, thus improving its buoyancy and enabling faster collection of the initial liquid volume. Moreover, the fluid pathway between the front side and the rear side of the stem allows the initial volume to be drained on both sides of the lower stem portion and into the collection receptacle. This has the advantageous effect of doubling the volume flow rate of the liquid during the collection of the initial volume, which again increases capture speed.

The present invention also relates to a kit of parts for assembling the liquid sampling device. The kit contains a casing that comprises a fluid inlet for receiving the liquid flow, a sample outlet for draining the initial volume of the liquid flow, a fluid outlet for draining a subsequent volume of the liquid flow, and a passageway for providing fluid communication between the fluid inlet and the fluid outlet and between the fluid inlet and the sample outlet. The kit further includes an inlet conduit connectable to the fluid inlet, an outlet conduit connectable to the fluid outlet, an elongated closure member slidably receivable by the casing, and a lifting member. The closure member has a head portion and a stem. The lifting member is a float connectable to or formed in the stem, and is adapted for moving the closure member from a sampling position to a diverting position while the initial volume of the liquid flow is being collected into a receptacle, connectable to the sample outlet of the casing. The head portion is adapted for obstructing the liquid flow through the fluid outlet when the closure member is slid into the sampling position. A lower portion of the stem, distal to the head portion, is adapted for obstructing the liquid flow through the sample outlet without obstructing the liquid flow through the fluid outlet when the closure member is slid into the diverting position. An upper portion of the stem, proximate to the head portion, comprises at least one longitudinal slit, which is stretching across the sample outlet to fluidly connect a front side of the stem facing the fluid inlet in the casing to an opposing rear side of the stem when the closure member is slid into the sampling position, thus allowing an initial volume of the liquid flow to exit said sample outlet via said at least one slit.

The kit may also comprise a collection receptacle and a cap for closing the collection receptacle. The collection receptacle may be a collector tube. The receptacle may be connectable to a sample connector of the liquid sampling device that is projecting outwardly from a base of the casing. The connection between the sample connector and the receptacle preferably is a threaded connection, in which, for instance, the receptacle comprises an external thread and the sample connector a matching internal thread. Those of skill in the art will appreciate that other connections, such as push-fit and snap-fit connections, can also be used. Instructions for use may be included in the kit.

According to embodiments of the invention, a sample connector of the casing is provided with internal threads to engage an externally threaded collection receptacle such as a collection tube in a threaded connection. This has the additional advantage that the full opening diameter of the collection receptacle is available for receiving the stem of the closure member, which improves the movement range of the closure member relative to the receptacle and allows for larger spaces between the inner walls of the collection receptacle and the closure member stem. As a result thereof, friction forces are reduced, as well as the risk of sticking and congestion by accumulation of the drained liquid during the capture process of the initial volume. In the diverting position of the closure member relative to the casing, the subsequent volume of the liquid can flow freely to the fluid outlet of the device through the slit(s) in the upper stem portion. This lowers the chance of mixing of the subsequent volume fraction, e.g. mid-stream urine, with the initial volume fraction that has been collected, e.g. first-void urine.

Embodiments of the invention allow collecting a small fraction of first-void urine in a standardized and volumetric manner, without the need of interrupting the urine flow. First-void urine, generally considered the first 20 mL to 50 mL of urine flow, contains higher concentrations of analytes associated with Human Papillomavirus (HPV) and Chlamydia trachomatis (CT) DNA than subsequent fractions. Additionally, first-void urine sampling is important to identify cancer biomarkers, such as prostate cancer.

The liquid sampling device according to embodiments of the invention is a user friendly and highly hygienic device that brings additional accuracy to urine-based testing in diagnostic application fields.

Embodiments of the invention allow obtaining a first-void urine sample as a valid, non-invasive sample by a self-sampling method that is adequate for all age groups and genders.

A further advantage of the present invention resides in the fact that narrow collection tubes with very small sample volume can be connected to the liquid sampling device and used for first-void urine capture. The improved buoyancy of the closure member and the significantly faster capture of the initial volume of liquid flow enables collection of first-void urine in collection tubes as small as 3 mL, or even less, e.g. between 1 mL and 3 mL, e.g. between 1.0 mL and 1.5 mL. Such small and narrow tubes can be used directly in liquid-handling robots for automated sampling, for instance in automated high-throughput analyzers. This assists with streamlining the pre-analytical process, shortening turnaround time, minimizing errors, as well as reducing costs. In addition thereto, the collector tubes may be pre-filled with a preservative for different urinary analytes, improving transport and storage of urine at room temperature.

Particular and preferred aspects of the invention are set out in the accompanying independent and dependent claims. Features from the dependent claims may be combined with features of the independent claims and with features of other dependent claims as appropriate and not merely as explicitly set out in the claims.

For purposes of summarizing the invention and the advantages achieved over the prior art, certain objects and advantages of the invention have been described herein above. Of course, it is to be understood that not necessarily all such objects or advantages may be achieved in accordance with any particular embodiment of the invention. Thus, for example, those skilled in the art will recognize that the invention may be embodied or carried out in a manner that achieves or optimizes one advantage or group of advantages as taught herein without necessarily achieving other objects or advantages as may be taught or suggested herein. The above and other aspects of the invention will be apparent from and elucidated with reference to the embodiment(s) described hereinafter.

### Brief description of the drawings

The invention will now be described further, by way of example, with reference to the accompanying drawings, in which:
FIG. 1 is a perspective view of a device for sampling an initial volume of a liquid flow according to an embodiment of the invention.
FIG. 2 and FIG. 3 are cutaway views of the liquid sampling device shown in FIG. 1 without a collection tube and with a connected collection tube, respectively.
FIG. 4 to FIG. 6 are rear, side and front elevation views of a closure member that can be used in embodiments of the invention.
FIG. 7 and FIG. 8 are perspective views of the closure member shown in FIG. 4.
FIG. 9 and FIG. 10 illustrate the fluid pathway through the device of FIG. 3 when the closure member of the device is slid to a sampling position and a diverting position, respectively.
FIG. 11 is an exploded view of a kit of parts for assembling a liquid sampling device according to the invention.

In the drawings, the size of some of the elements may be exaggerated and not drawn on scale for illustrative purposes. The dimensions and the relative dimensions do not necessarily correspond to actual reductions to practice of the invention. Any reference signs in the claims shall not be construed as limiting the scope. In the different drawings, the same reference signs refer to the same or analogous elements.

### Detailed description of illustrative embodiments

The present invention will be described with respect to particular embodiments and with reference to certain drawings but the invention is not limited thereto but only by the claims.

The terms first, second and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art.

It should be noted that the use of particular terminology when describing certain features or aspects of the invention should not be taken to imply that the terminology is being re-defined herein to be restricted to include any specific characteristics of the features or aspects of the invention with which that terminology is associated.

In the description provided herein, numerous specific details are set forth. However, it is understood that embodiments of the invention may be practiced without these specific details. In other instances, well-known methods, structures and techniques have not been shown in detail in order not to obscure an understanding of this description.

In the context of the present invention, a liquid to be sampled typically relates to urine. However, a liquid sampling device according to the invention is suitable for sampling liquids in general, including other bodily fluids, water, etc.

When reference is made in embodiments of the invention to a first-void volume of urine, this generally designates the first 20 mL to 50 mL of the initial urine flush. Small volumes in this respect, refer to volumes (e.g. first-void volume) which are smaller than 20 mL, e.g. less than 10 mL, e.g. less than 5 mL, such as for instance 4 mL. Very small volumes refer to volumes (e.g. first-void urine) less than or equal to 3 mL, e.g. between 1 mL and 3 mL, e.g. between 1.5 mL and 3 mL, e.g. between 2 mL and 3 mL, e.g. between 2.5 mL and 3.0 mL, e.g. between 2.6 mL and 2.9 mL.

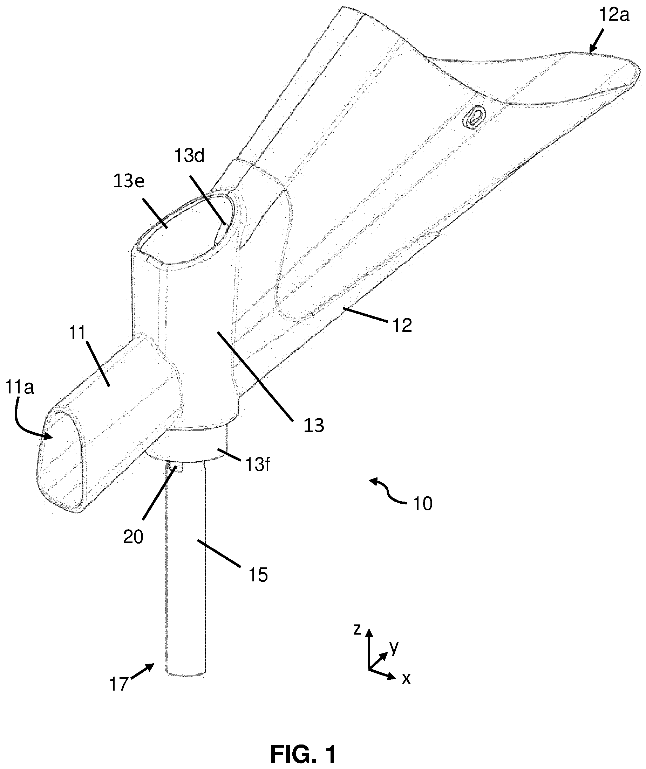

With reference to FIG. 1 to FIG. 3 of the drawings, a device for sampling an initial volume of a liquid flow according to an embodiment of the invention is now described. The sampling device 10, which is shown in perspective view in FIG. 1, comprises an inlet conduit 12, an outlet conduit 11, a casing 13 and an elongated closure member 17 that is removably insertable into the casing via a an open-ended guiding structure 13e in the casing, e.g. a guiding tube, channel or shaft.

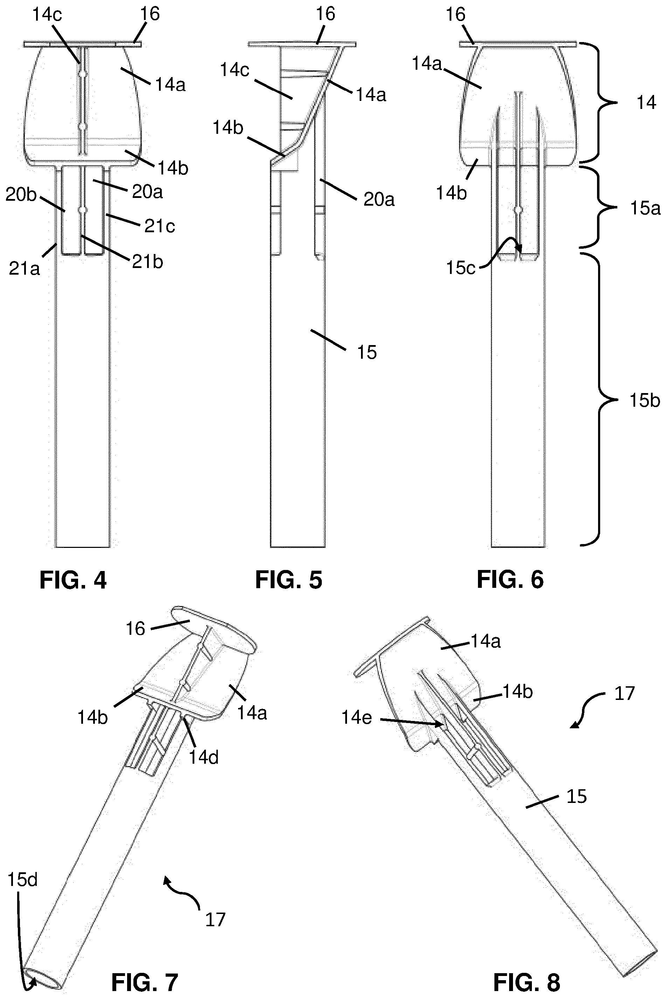

The closure member 17, is slidably supported in the casing and can be displaced axially along the longitudinal direction of elongation 'z' of the closure member, relative to the casing. The guiding structure 13e restricts the movement of the closure member 17 to only slide along the vertical axis 'z'. A resilient protrusion 13d is formed on an inner surface of the guiding structure 13e and acts as a stopping element, e.g. limits the movement of the closure member when it is slid upwards in the casing. A base section 13f of the casing 13 limits the axial displacement of the closure member 17 when it is slid downwards in the casing. The protrusion 13d and the base 13f respectively define a sampling position and a diverting position of the closure member relative to the casing. When inserted into the casing 13, a stem 15 of the closure member 17 is extending vertically through an opening in the base 13f. The circumferential edge of the stem 15 and the opening in the base have corresponding shapes so that the opening assists and stabilizes the sliding movement of the closure member relative to the casing. At least one longitudinally extending slit 20 (only partially visible in FIG. 1) is provided in an upper portion of the stem 15. The at least one slit stretches across the opening in the base 13f and opens into the interior of the casing when the closure member is moved into the sampling position. The closure member will be further described in relation to FIG. 4 through FIG. 8.

The inlet conduit 12 is adapted for receiving a liquid flow, e.g. urine, at a distant opening 12a and for guiding it towards the casing 13 and the outlet conduit 11 is adapted for draining the subsequent volume of the liquid flow, e.g. urine, away from the casing 13 and for expelling it from the device 10 at a distant opening 11a. Although the inlet conduit 12 and the opening 12a can be shaped in numerous ways, an inlet conduit 12 that forms a funnel is preferred for the purpose of collecting the liquid efficiently and without spillage. A funnel-shaped inlet conduit 12 with a widened receiving lip as opening 12a is particularly advantageous for the sampling of urine, since it offers the user a more hygienic and more comfortable use of the device 10. Moreover, the inlet conduit 12 and the outlet conduit 11 are preferably arranged at a slanting position with respect to a vertical z-axis along which the elongated closure member 17 is typically aligned during operation of the device 10, e.g. at angles ranging between 20° and 70°, e.g. about 45°. This has the advantage that a liquid flow which enters the device 10 at the opening 12a at a low flow velocity is forced onward without delay, whereby a faster sampling of the initial volume thereof is achieved while contamination and sticking of the liquid to the inlet conduit 12 walls is prevented. Similarly, the removal of the subsequent volume of the received liquid flow from the casing 13 is accelerated by the slanted outlet conduit 11, thereby reducing the risk of contaminating the sampled initial volume by residuals of the subsequent volume remaining in the casing 13.

The outlet conduit 11 may be adapted for connection to a further receptacle for capturing the subsequent volume, or may be adapted for connection to a further liquid sampling device, e.g. a second sampling device in accordance with embodiments described hereinabove (and suitable outlet to inlet connectors) for sampling a fraction of the subsequent volume of the liquid flow, e.g. a fraction of the mid-stream urine.

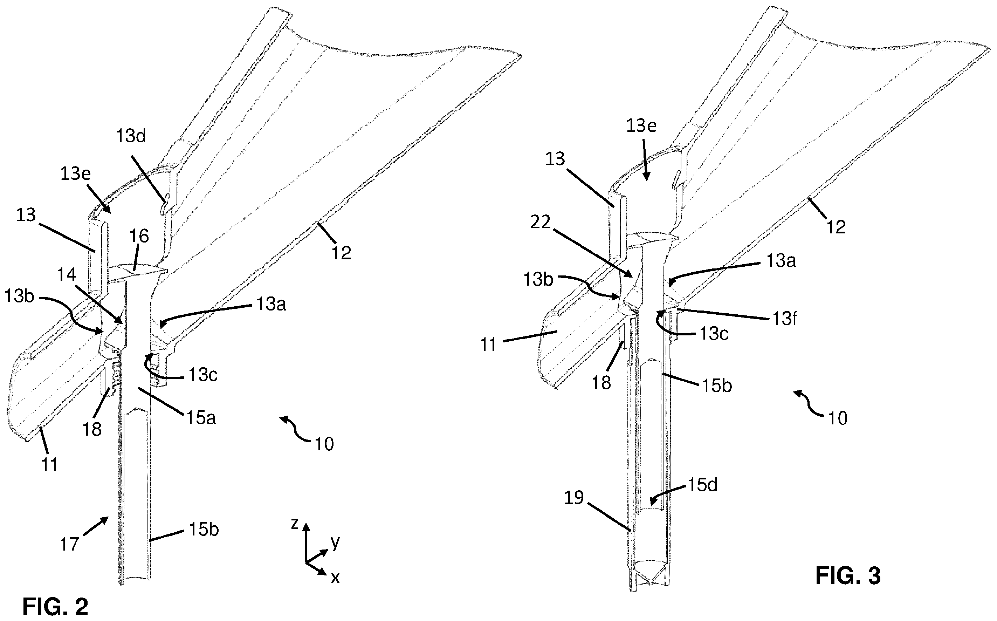

Turning now to FIG. 2, the interior of the sampling device 10 of FIG. 1 is further explained in a cutaway view. The cutting plane is taken as the mirror plane of the device (y-z-plane), perpendicular to the x-axis.

As illustrated in FIG. 2, the casing 13 comprises a projecting side wall or projecting side walls (in the z-direction) that have their lower sides supported by the flat base 13f. The projecting side wall(s) define a hollow interior, e.g. a channel or duct, which is used as the guiding structure 13e for the closure member 17. Internal surfaces of the channel are shaped to receive and slidably support a head portion 14 of the closure member, which is wider than the stem 15. More specifically, a terminal section or cap 16 of the head portion 14 has an outer edge or rim that is form-fitted with the internal surface of the channel. In other words, the guiding structure 13e acts as a sliding bearing vis-à-vis the wider head portion 14, which is an extension of the thinner stem of the closure member. An internal surface or surfaces of the guiding structure 13e, e.g. internal surface of the vertical channel or duct formed inside the casing 13, corresponds to the slide, i.e. the surface on which the head portion 14 of the closure member is supported and slides.

In the inserted configuration shown in FIG. 2, the wider head portion 14 is retained, at its bottom side, by the base 13f of casing 13. The inward face of the base 13f is a bearing surface in respect of the head portion 14. When the closure member is slid, e.g. moved from the sampling position, where the head portion 14 rests on the interior face of the base 13f, to the diverting position, where the cap 16 abuts on the protrusion 13d, the head portion 14 remains enclosed by the projecting side walls of the casing, whereas the upper portion 15a of the stem retracts into the casing 13 via an opening in the base 13f. This opening also functions as a sample outlet 13c through which the initial volume of the liquid flow is being collected: The sample outlet 13c, e.g. a circular opening, is formed through the base of casing 13 and reaches into a passageway 22 that is extending between a fluid inlet 13a and a fluid outlet 13b and through the hollow interior of the casing 13. Depending on whether the closure member 17 is moved into the sampling position or the diverting position, only the sample outlet 13c or only the fluid outlet 13b is selected to be in fluid communication with the fluid inlet 13a. The selection mechanism and the corresponding liquid sampling process are explained further below.

The free play between the sample outlet 13c and the lower portion 15b of the stem 15 is small enough to establish a liquid seal when the closure member is slid into the diverting position and the lower stem portion 15b obstructs the sample outlet 13c, but still allows good mechanical sliding of the stem relative to the sample outlet. Fluid sealing is required to prevent mixing and contamination between the collected initial volume and the subsequent volume of the liquid flow that is diverted through the fluid outlet 13b. Minimal frictional forces between the stem and the sample outlet preferably are preferred to achieve a quick lifting of the closure member while the initial volume of the liquid flow is being collected.

In addition to the sample outlet 13c, the fluid inlet 13a and fluid outlet 13b are formed as openings in the projecting side walls of the casing 13. The inlet conduit 12 and the outlet conduit 11, respectively, connect to the fluid inlet 13a and the fluid outlet 13b. The end faces of the inlet conduit 12 and the outlet conduit 11 that are proximate to the casing 13 may be of equal shape and size as the fluid inlet 13a and the outlet 13b respectively, or may be shaped differently and/or differ in size. In this particular embodiment, a permanent alignment of the inlet conduit 12, the outlet conduit 11, and the fluid inlet and outlet of the casing 13 has been achieved, e.g. by providing the conduits 11, 12 as integral parts of the casing 13. In other words, the conduits 11, 12 and the casing 13 constitute a single, monolithically formed unit, in which the each conduit 11, 12 is directly connected to the projecting side wall of the casing. This way of connecting the conduits 11,12 to the casing 13 is not limiting; alternative means for connecting the conduits to the casing may be provided instead. For instance, a snap-fit connection may be made between plastic pieces, a threaded connection, or push-in fittings or compression fittings may be used to connect the casing 13 to soft or hard tubing used for the conduits 11, 12.

For the embodiment presently described, the sliding axial movement of the closure member 17 corresponds to a linear movement along the vertical direction (e.g. z-axis), which is transverse to the direction of liquid flow through the passageway between fluid inlet 13a and fluid outlet 13b, e.g. in a direction substantially perpendicular to the direction of flow through the passageway (e.g. y-axis). Notwithstanding the preferred 90° angle between the axial movement of the closure member 17 and the direction of flow through the passageway, e.g. as defined by an inclination angle of the flat base of the casing relative to the z-axis, embodiments of the invention are not limited to a 90° angle or angles close to 90°. For instance, the flow direction for an inclined base may be slanted and the resulting angle between the flow direction through the passageway and the axial movement of the closure member 17 may take values in the range from 65° to 90°.

The sampling device further comprises a lifting member that is adapted for moving the closure member 17 from the sampling position into the diverting position while the initial volume of the liquid flow is being collected through the sample outlet 13c, e.g. into a receptacle that is connectable to the base of the casing. The lifting member is executed as a float that is formed inside or attached to the lower stem portion 15b. A buoyancy force is acting on the float whilst the initial volume is being collected into a receptacle that encloses the float. In the present embodiment, the float corresponds to an elongated air-filled cavity (e.g. air pocket) 15 d in the lower portion 15b of the stem 15. The air-filled cavity has an aperture at the bottom side of the stem 15 and extends longitudinally into the lower stem portion 15b. However, different embodiments of the invention may be provided with a different lifting member that has floating ability, for instance a block connected to an end portion of the stem or a block fastened to the stem and surrounding the same, which block comprises one or more air pockets, air-filled cavities, or comprises a porous material, e.g. a foam (e.g. an extruded polystyrene foam).

FIG. 3 corresponds to the cutaway view in FIG. 2 with the addition that a collection tube 19 is shown in a connected state with respect to the casing. The collection tube is a particular example of a receptacle for collecting the initial volume of the liquid flow. A connector 18 with downwardly projecting walls is arranged on the outward face of the base 13f of the casing 13 so that the connector walls encircle the sample outlet 13c. The connector walls comprise an internal thread that engages a corresponding outer thread on the collection tube 19 to form a threaded connection and releasably secure the collection tube to the casing. A benefit of using an externally threaded collection tube or receptacle, e.g. compared to a collection tube that is push-fitted onto a sample connector, is that the closure member stem can take advantage of the full opening of the tube, since the internal diameter of the tube is fully available for receiving the stem and not reduced due to internal threading on the tube. This allows for larger gaps, e.g. 1 mm or more, and reduced flow constriction in the region between the lower stem portion and the inner walls of the collection tube. Surface tension effects and capillary action in this area, which slow the lifting of the closure member, are also eased. The resulting improved flow properties of the liquid thus contribute to the accelerated capture of the initial volume and the reduced risk of mixing between the initial volume to be collected and the subsequent volume of the liquid flow.

A closure member that can be used in a liquid sampling device according to the invention is now described in more detail with reference to FIG. 4 through FIG. 8, of which FIG. 4 shows the closure member in rear elevation, FIG. 5 in side elevation and FIG. 6 in front elevation. FIG. 7 and FIG. 8 are corresponding perspective views of the closure member. Here, a front side of the closure member refers to the side that is facing the fluid inlet 13a when the closure member is inserted into the casing. Likewise, a rear side of the closure member is defined as the side which is facing the fluid outlet 13b when the closure member is inserted into the casing. The numerous details presented in respect of the closure member help elucidating its role in selectively blocking the liquid flow either through the fluid outlet or the sample outlet.

The head portion 14 of the closure member 17 is configured to obstruct the liquid flow through the fluid outlet 13b whilst the initial volume of the liquid to be sampled flows from the fluid inlet 13a to the sample outlet 13c and into a receptacle connectable to the casing, i.e. when the closure member is moved into the sampling position with respect to the casing. A slanted barrier wall of the head portion 14, preferably composed of two flat and differently sloped wall segments 14a and 14b, achieves this obstruction effect. In the sampling position of the closure member, the head portion 14 is positioned inside the passageway 22 and a bottom edge of the barrier wall, e.g. the bottom edge of the second wall segment 14b, is resting on the inward face of the casing base 13f in a region that is not overlapping the sample outlet 13c. In that case the rim of the base 13f surrounding the sample outlet 13c acts as a seat for the slanted barrier wall of the head portion. A farther movement of the closure member 17 downwards with respect to the stationary casing 13 is thereby prevented.

Moreover, lateral edges of the first and second flat wall segment 14a, 14b are form-fitted with respect to the inner surface of the guiding structure 13e such that the passageway 22 is sealed off and the initial volume of the liquid flow is prevented from exiting the passageway through the fluid outlet. Accordingly, the flat wall segments 14a, 14b of the head portion behave like the gate of a valve that can moved into and out of the passageway. The bottom edge of the second wall segment 14b extends, smoothly and without interruption, in a lateral direction (e.g. x-direction) and spans a distance that is wider than the stem 15 and wider than the sample outlet 13c in the base 13f of the casing 13. The contour of the slanted barrier wall is continuous and uninterrupted and may take the form of the blade of a spade that has curved side edges and a straight edge at its top, e.g. where the cap 16 connects to the first wall segment 14a. Preferably, the contour of the slanted barrier wall, when projected onto a frontal plane, encompasses the contour of the fluid inlet 13a when projected onto the same frontal plane.

The front side of the first wall segment 14a and an upper portion of the second wall segment 14b, proximate to the first wall segment 14a and distal to the bottom edge of the second wall segment 14b, may be partitioned by a thin vertical wall 14c that contacts and supports the cap 16. This additional vertical wall 14c has the advantage of increasing the structural integrity of the closure member 17, in particular of the cap 16 during its sliding contact with the casing. The first and second flat wall segments 14a, 14b are continuous with each other and form an extension of the stem 15. A transition between the first flat wall segment 14a and the second flat wall segment 14b is located half-way along the stem diameter (e.g. in y-direction). Accordingly, the first wall segment 14a rises upwards on the front side of the stem, whereas the second wall segment 14b slopes downwards on the rear side. The inclination angle of the second wall segment 14b is steeper than the inclination angle of the first wall segment 14a, wherein the inclination angle of the flat wall segments 14a, 14b is measured relative to the longitudinal axis of the elongated closure member (e.g. z-axis). Sloping wall segments have the advantage that they efficiently redirect the initial volume of the liquid to flow in a substantially vertical direction, although the original flow direction of the liquid entering the passageway through the fluid inlet is substantially horizontal. A further advantage of the sloping wall segments 14a, 14b is given by the fact that the received initial volume of the liquid flow is exerting a (dynamic) pressure force on the wall segments 14a, 14b. This pressure force has a component parallel to the flow direction and an upward component parallel to the z-axis. Therefore, inclined wall segments 14a, 14b can be used to further enhance an upwards directed lifting force, in addition to the buoyancy force exerted on the lifting member, which results in an even faster sliding of the closure member from the sampling position to the diverting position. Providing a slanted barrier wall that is a composite wall including differently sloping flat wall segments 14a, 14b has proved to result in better flow redirection capability and improved secondary lifting force. The second flat wall segment angled wings on the redesigned floater allow for a better guided flow towards the collection tube/receptacle and generate additional lifting effects.

The secondary lifting force is much appreciated in cases of small sampled initial volumes, e.g. less than 4 mL of first-void urine, e.g. between 2mL and 3mL, for which a volume of the lifting member that is submerged in the already sampled and collected liquid is typically small. Very small-volume collection tubes, e.g. between 2 mL and 3mL, are particularly affected by the reduced buoyancy forces. Narrowing the diameter of these tubes is problematic as capillary adhesion forces will become decisive in the constricted flow region between the stem and the edge of the sample outlet. The adhesion forces may delay and hamper the collection process of the initial volume and the risk of contaminating the initial volume with the subsequent volume increases, e.g. contamination of an earlier fraction of the first-void urine by a later fraction of first-void or mid-stream urine. The secondary lifting force can mitigate this effect to some extent.

In some embodiments of the invention, similar to the one shown in FIG. 7, one or more ribs 14d may be arranged on the front side of the second flat wall segment 14b. The ribs 14d connect the second flat wall segment 14b to the circumference of the upper stem portion 15a and structurally strengthen the flat wall segment 14b. The ribs 14d may in particular reduce vibrations and/or the risk of damage of the second wall segment 14b. In such embodiments, the ribs 14d enter into physical contact with the interior face of the base 13f of the casing 13 when the closure member is moved into the sampling position. To reduce a gap between the bottom edge of the second wall segment 14b and the interior face of the base 13f of the casing 13 in the sampling position, ribs 14d with minimal height at the bottom edge of the second wall segment 14b are preferred. The presence of the ribs reduces the contact surface between the head portion and the base of the casing when the closure member is in the sampling position. This has the advantage that the risk of the head portion sticking to the base is reduced, allowing a swift lifting of the head portion and stem as soon as the collection of the initial volume of the liquid flow starts. Manufacturability is another advantage of the ribs 14d. If ribs 14d would not be present, a mold, for instance a steel mold, would have to have a very sharp edge to be able to mold the second wall segment 14b. This could lead to weakness and fast wear and tear of the mold. Additionally, without the ribs 14d, the part cannot be adequately de-molded. Therefore, ribs 14d ensure that the injection mold has a longer life span.

The head portion of the closure member is terminated by a flat top section or cap 16. It is an advantage of embodiments of the invention that the cross-section of the cap 16 (in planar view, perpendicular to the z-axis) can be fitted to match, in shape and size, the inner boundary of the guide channel 13e in the casing 13 such that the sliding axial movement of the closure member relative to the stationary casing is assisted by the cap 16. The cap 16 is slidably supported on the inner surface of the guide channel 13e, which acts as a sliding bearing for the cap 16, as well as for the lateral edges of the slanted barrier wall. The cap 16 may be provided as an asymmetrically-shaped disk, e.g. an originally circular disk of which a segment has been removed along a chord. An asymmetrically shaped cap 16 that is fitted to the cross-sectional shape (e.g. in x-y-plane) of the guide channel 13e has the advantage that a rotation of the head portion and closure member as a whole around the vertical axis of displacement is prevented. Other shape combinations for the cap and the guide channel can be selected to yield the same effect, e.g. elliptical shape, polygonal shape, etc.

It is noted that the guiding aspect can be further improved by fitting the circumference of the stem 15 in the x-y-plane to the inner edge of the sample outlet 13c, thus creating another sliding bearing for the stem 15 of the closure member. As a result of the improved guiding of the closure member 17 at two different height levels (e.g. different positions in z-direction), tilting of the closure member 17 can be prevented. In addition thereto, the so fitted cap 16 provides enhanced sealing of the passageway 22 relative to the part of the guiding structure 13e that is located above the cap. Splashes or spilling of the initial and subsequent volume of the received liquid flow, e.g. urine, out of the upper opening of the guiding structure can thus be prevented. The skilled artisan knows, by design routine or trial and error, how to select appropriate clearances between the cap and the inner surface of the guiding structure, between the contours of the slanted barrier wall and the inner surface of the guiding structure, and between the stem and the sample outlet such that the guiding is performed without significant friction and yet amenable to sealing off the passageway. The skilled person is also aware that a small clearance may be needed and is permissible to move the closure member 17 back into the sampling position after the liquid flow has ceased and the sampled initial volume securely removed.

The stem 15 of the closure member 17 comprises a lower stem portion 15b and an upper stem portion 15a. The lower stem portion 15b is continuously connected to the upper stem portion 15a, e.g. the transition between the outer surfaces of both stem portions is smooth and without a change in shape or size (i.e. same circumference). An air-filled cavity 15d is provided centrally inside the lower stem portion 15b such that an aperture of the cavity 15d is situated on the bottom surface of the stem 15. Hence, the lower stem portion 15b may take the form of a half-open hollow cylinder. The air-filled cavity 15d inside the stem 15 acts as a float in respect of the initial volume of the liquid that is collected into a receptacle such that the lower stem portion 15b is at least partly submerged.

During use of the sampling device, the liquid flow pertaining to the initial volume is sampled through the sample outlet 13c and generates a liquid column in a receptacle that has been previously connected to the device, e.g. to the sampling connector 18. Preferably, the shape and depth of the receptacle is selected to cooperate with the float that formed inside or connected to the lower stem portion 15b in that the formation of the liquid column in the receptacle is accompanied by a quickly progressing immersion of the float. A float in the form of an air-filled cavity 15d that is arranged in the hollow interior of the lower portion of the stem has the additional advantage that material weight and cost relating to the formation of the elongated stem is conveniently saved by provision of the cheap, low density fluid air. This also allows connectable receptacles of smaller diameter to be used, which efficiently increase the immersed volume of the float, hence to increase the buoyancy force. Nevertheless, embodiments of the invention are not limited thereto and other floating structures and/or materials may be used instead of, or in addition to, air cavities in the lower portion of the stem, for instance a block of foam into which the lower end portion of the stem extends. As a consequence of the buoyancy force acting on the lifting member as a float, the closure member as a whole is lifted and forced upwards as the height of the sampled liquid column continues raising. The buoyancy-driven vertical displacement is stopped, and a further elevation of the closure member prevented, as soon as the diverting position is adopted.

The upper stem portion 15a is provided as an open structure and comprises at least one longitudinal slit, e.g. two longitudinal slits 20a, 20b as shown in FIG. 4. The longitudinal slits create a diametrically extending fluid pathway through the upper stem portion 15a, meaning that opposite sides of the stem 15 are fluidly connected through an aperture in the upper stem portion 15a. In the context of the present invention, the wide slits are preferred to avoid capillary forces and adhesion effects. Therefore, the slits may also be referred to as slots. It can be understood from FIG.4, FIG. 5 and FIG. 6 that the elongated slits 20a, 20b establish fluid communication between the front side and the rear side of the stem 15. The longitudinal slits divide the upper stem portion 15a into a plurality of vertical support sections or vertical support columns 21a-21c that contact the flat wall segments 14a, 14b at their top. The central, upward raising vertical support section 21b structurally strengthens the upper stem portion 15a and minimizes the risk of breaking or bending the stem. The vertical support sections 21a, 21b, 21c reduce, possibly even minimize, surface tension. As the vertical support sections 21a-21c of the upper stem portion 15a abut directly on the front side of the first and second wall segment 14a, 14b, an upper side of the slits 20a, 20b is delimited by the front side of the slanted barrier wall. In the region where the slits 20a, 20b contact the front side of the wall segment 14a, 14b, gradually narrowing flow channels 14e are formed along which the redirected liquid is being drained towards the slits and the sample outlet. At their lower side, the slits 20a, 20b are delimited by a flat surface 15c, which is a partially exposed surface at the interface between the lower and the upper portion of the stem, and which also aids in reducing surface tension.

The slits extend downwards in the longitudinal direction of the closure member. Their smallest longitudinal dimension is larger than the thickness of the sample outlet 13c (e.g. length in z-direction) through the base 13f of the casing such that each slit stretches across the sample outlet when the closure member is moved into the sampling position. In consequence, the elongated slits also provide a fluid connection between the passageway 22 and the outer surface of the lower stem portion 15b when the closure member is in the sampling position relative to the casing. Therefore, the initial volume of the liquid flow can enter the passageway through the fluid inlet and exit the passageway through the elongated slits in the upper stem portion. Gravity accelerates the redirected liquid while falling downwards in the slits.

During use of the sampling device, a receptacle for collecting the initial volume is connected to the casing such that the lower stem portion reaches into the receptacle. A gap between the walls of the receptacle and the outer surface of the lower stem portion 15b allows the initial volume of the liquid flow to reach the bottom of the receptacle. As the entrance area of the slits in the front elevation of FIG. 6 is significant, e.g. larger than the area associated with the vertical support sections in the same front elevation, the draining and collection process of the initial volume into the receptacle can be carried out very quickly. In other words, the total width of the slits in y-direction can be made almost as large as the diameter of the stem so that high flow rates of the liquid through the slits and into the receptacle are feasible. The inclined first and second flat wall segments 14a, 14b cooperate with the slits in redirecting the received liquid stream from a horizontal or nearly horizontal flow direction onto a substantially vertical flow direction as long as the initial volume is being collected.

In contrast to the open structure of the upper stem portion 15a, the lower stem portion 15b has a closed structure without orifices through which the liquid could flow. When the closure member is moved into the diverting position, the lower stem portion 15b extends through the sample outlet 13c and the upper stem portion 15a is positioned inside the passageway 22. The circumference of the lower stem portion being form-fitted to the shape of the sample outlet, the lower stem portion effectively provides a plug that seals off the sample outlet. Therefore, the subsequent volume of the liquid flow is prevented from exiting the passageway through the sample outlet. As the head portion of the closure member has been lifted from the base of the casing when the closure member is moved into the diverting position, and the upper stem portion is thinner than the slanted barrier wall of the head portion and also contains slits, the subsequent volume of the liquid flow is able to traverse the passageway and reach the fluid outlet without major obstacles. Therefore, the subsequent volume of the liquid flow is diverted through the fluid outlet when the closure member is moved to the diverting position.

Compared to the closure member described in the prior art reference WO 2021/069454 A2, which teaches a closed central structure in the form of half-pipe, the closure member according to embodiments of the present invention has an open structure in the upper stem portion. The slits in the upper stem portion reach through the stem and fluidly connect the front side of the stem with its rear side. This has two benefits: weight reduction and improved flow guidance. Indeed, an open structure requires less material, e.g. less plastic material in an injection molding process, which contributes to a more efficient and cheaper manufacturing of the liquid sampling device. A lightweight closure member is also lifted more rapidly by the float, which guarantees a faster capture of the initial volume. Especially for very small volumes of first-void urine, e.g. less than 3 mL, fast capture is advantageous in terms of accuracy of the sampled volume and quality of the captured sample (e.g. less risk of contamination by mid-stream urine). Weight reduction also assists with achieving device miniaturization, e.g. for the purpose of capturing only very small volumes. It is observed that weight reduction of the state-of-the-art closure member in WO 2021/069454 A2 by a reduction in the wall thickness is very limited, because injection molding processes strictly require a minimum wall thickness, e.g. 0.5 mm.

Moreover, replacing the conic area of the beveled transition segment of the state-of-the-art closure member by a flat surface 15c leads to a further reduction in weight. The open structure of the present closure member permits larger volume flow rates and facilitates removal of the liquid on both the front and the rear side of the lower stem portion. This means that a beveled transition segment would only have little or no effect on the capture process of the initial volume and can be eliminated. Besides, the longitudinal slits can be designed long enough to avoid that the redirected liquid is projected back into the casing.

With regard to the improved flow guidance property of the slotted upper portion of the stem, it is noted that the existence of an open central structure strongly reduces the lateral forces that are exerted by the liquid flow on the upper stem portion. These lateral forces are particularly pronounced if the sampling device is used by people that have high urination rates, e.g. 40 mL/second or higher. Removal of the closed central structure (grooved upper stem portion) in the state-of-the-art closure member, reduces the area on which the lateral forces can act. It also leads to a more efficient operation of the device as excessive lateral force has the potential to slow or block the movement of the float due to friction.

Furthermore, the subsequent volume of the liquid (e.g. mid-stream urine) passes freely to the fluid outlet of the device and is also flowing through the slits in the upper stem portion. This increase in the available cross-sectional flow area (e.g. in the x-z-plane) inside the passageway 22 lowers the chance of mixing the subsequent volume of the liquid with the initial volume (e.g. first-void urine) that has already been collected.

In applications in which only a very small volume of the liquid is to be collected (e.g. 2-3 mL), for instance when small-volume collection tubes are used in subsequent diagnostics, there is only a very little amount of liquid available that can generate buoyancy. At an average urination rate of 16 mL/second, there is also very little time for the closure member to slide from the sampling position to the diverting position to ensure that only the initial volume of liquid flow is sampled. The open central structure of the present closure member allows for liquids to flow down both sides of the lower stem portion during the capturing step of the initial volume. This increases, e.g. doubles, the rate at which the sample can be collected in a collection tube or receptacle.

The absence of the closed central structure in closure members of the present invention allows for the first and second flat wall segments 14a, 14b to cover a large surface area that receives and interacts with the liquid flow. The shape of the first and second flat wall segments 14a, 14b provides a reduced weight of the closure member, so as to enhance its buoyancy. The front surface of the first and second flat wall segments 14a, 14b can be uninterrupted, e.g. fully formed and stretching across the entire width (e.g. in the x-direction) of the head portion inside the passageway of the casing. As a result thereof, the secondary lifting force can be greatly increased, thereby improving the floating capability of the closure member as a whole and reducing the time for sliding the closure member from the sampling position into the diverting position. Additionally, providing the head portion with the continuous, broadened wall segments 14a, 14b leads to a better control of the flow properties of the liquid inside the passageway of the casing.

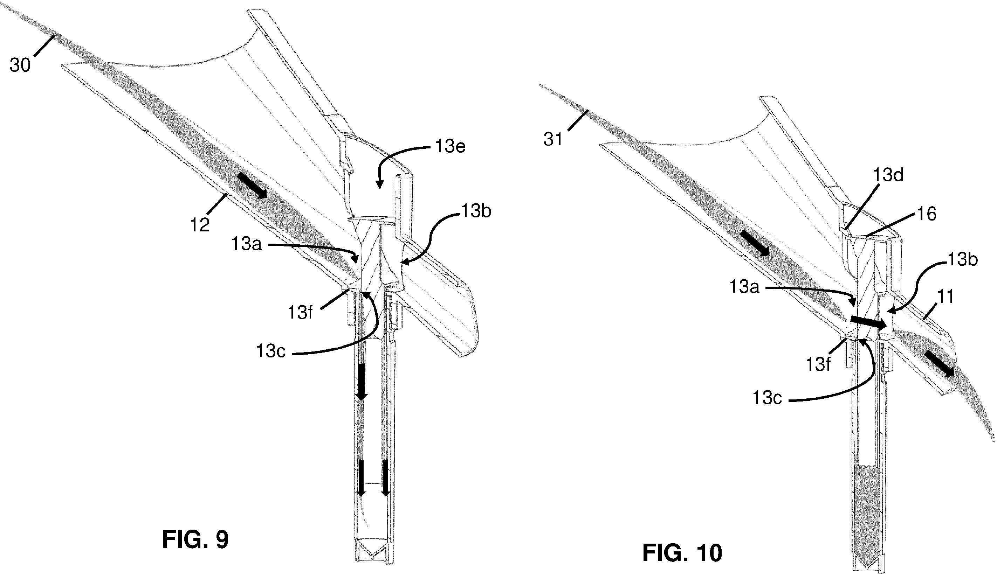

The following describes a best mode of operating the device for sampling an initial volume of a liquid flow according to embodiments of the invention. Reference will be made to FIG. 9 and FIG. 10, which depict the pathway of the liquid, e.g. urine, as it flows through the liquid sampling device when its closure member is moved into the sampling position and diverting position respectively.

To detect sexually transmitted infections in urine samples, samples taken from of a first small fraction of first-void urine, e.g. the initial 4 mL thereof, are recommended; this improves the reliability of the results obtained by urinalysis. Collecting an even smaller volume of first-void urine, e.g. less than the initial 4 mL, e.g. less than 3 mL, e.g. between 2 mL and 3 mL, e.g. between 2.6 and 2.9 mL, has the additional advantage of improving the quality of the sample for diagnostic purposes. Therefore, care has to be taken to not spill or lose any of the initial volume during the process of taking samples. This should not come at the expense of less handling comfort and hygiene when using the sampling device. Prior to sampling the initial volume of the liquid, e.g. urine, the user of the sampling device verifies that the closure member is positioned in the sampling position relative to the device casing. The user can do so by visually inspecting the stem of the closure member to check that the lower portion of the elongated slits are positioned outside the casing, e.g. as shown in FIG. 1. Next, the user connects a suitable receptacle, such as a collection tube, to the casing or, if the receptacle has been previously connected to the valve casing, may check that it is connected in a secure way, e.g. such that it cannot loosen and fall down subsequently. Preferably, the receptacle is secured to a sample connector of the casing by means of a threaded connection, e.g. by screwing an externally threaded collection tube into an internally threaded sample connector. Alternatively, the connection may be established by snap-fitting or push-fitting the receptacle onto a sample connector. In cases, in which the sampling device is delivered to the user as a kit of parts, optionally including further parts such as expandable funnels, the user is prompted to assemble these parts as described hereinabove. A buffer solution or preservation agent may be added to the bottom of the receptacle. The user then positions the distant opening 12a of the inlet conduit 12 on the urinary meatus, wherein the wide funnel-shaped opening 12a is adapted to the anatomical shape around the meatus to prevent spillage or losses during the time urine is being execrated by the user's body and received by the inlet conduit 12. At the same time, the user is positioning the distant opening 11a of the outlet conduit 11 over or into a further recipient, e.g. into a larger container or a toilet. Next the user verifies that the sampling device is oriented nearly vertically and starts the urination process.

An initial volume of first-void urine 30 enters the sampling device via the inlet conduit 12, passes through the fluid inlet 13a and encounters the slanted barrier wall of the head portion that is obstructing the fluid outlet 13b. The slanted barrier wall diverts the flow of first-void urine into a vertical direction, the diverted flow passes through the longitudinal slits that stretch across the sampling outlet 13c, thus leaving the casing. Eventually, the initial volume of first-void urine 30 flows through the gap between the outer wall of the lower stem portion and the inner wall of the receptacle and is collected at the bottom of the receptacle. It is worth noting that the slits allow the initial volume of the liquid flow to access and traverse this gap on both sides of the lower stem portion, e.g. on the front side and the rear side. This has the advantageous effect that the volume flow rate can be doubled as compared to a single-sided collection mechanism. The float therefore reacts much faster and very small initial volumes of first-void urine, e.g. smaller than 3 mL, e.g. about 2 mL, can be collected accurately and without contamination by the subsequent volume.

While receiving the initial volume of the liquid flow, the lifting member (i.e. float) forces the closure member upwards until the cap 16 is pushing against the protrusion or stopper element 13d. At this moment in time, the closure member has been slid into the diverting position and collection of the initial volume of urine has been completed. The sample outlet 13c is now being obstructed by the lower portion of the stem. However, the thinner diameter of the stem compared to the head portion and the slits that pierce the upper stem portion permit the subsequent volume of urine 31 to flow through the passageway in the casing 13, out of the fluid outlet 13b and towards the distant opening of the outlet conduit 11, where it is expelled from the sampling device. Once the urination is completed and no more urine is entering the inlet conduit 12, the user loosens the receptacle with the sampled initial volume. A closing lid can be firmly screwed or snapped onto the receptacle for safe delivery of the urine sample to the laboratory charged with the urinalysis and the detection of pathogens. The user may further dispose of the sampling device, e.g. by flushing a biodegradable device in the toilet, or may wash or rinse and disinfect the sampling device for a next use (optionally replacing accessories such as funnels).

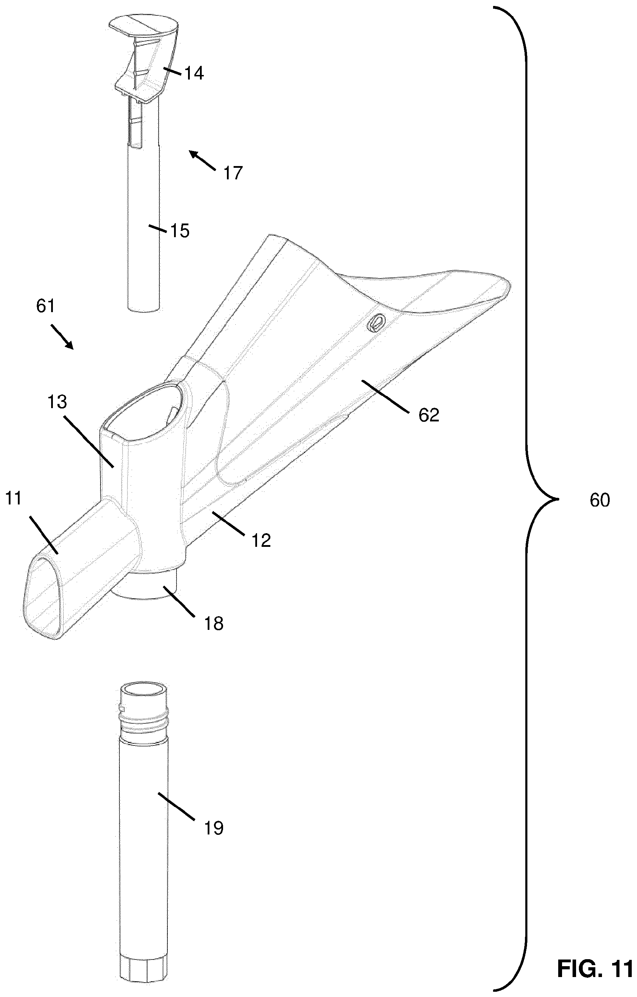

Components of the device for sampling an initial volume of a liquid flow as described hereinabove, as well as attachable accessories, may be provided separately in a kit of parts. With reference to FIG. 11, an exploded view of such a kit 60 is shown. The kit of parts 60 is an assembly kit for the sampling device of FIG. 1 and additionally contains a sample collection tube 19 that can be threadedly secured to a sample connector 18 of the casing 13. It is an advantage of the kit 60 that the parts can be manufactured separately before they are delivered to the user as a complete kit in a compact box (e.g. smaller than e.g. 380 mm x 265 mm x 32 mm, and shipped by regular mail), or as replacement parts for a previously purchased kit, who is assembling the sampling device according to instructions.

The kit of parts 60 comprises the closure member 17, the inlet conduit 12, the outlet conduit 11, the casing 13, and an optional receptacle such as the collection tube 19. Preferably, the casing 13 and the inlet and outlet conduits 12, 11 are integral parts of a single monolithically formed unit 61 such that both conduits 11, 12 are permanently connected to the casing 13 and openings of the conduits proximal to the casing coincide with the fluid inlet and fluid outlet of the casing. The receptacle 19 (preferably with closing lid) is connectable to a sample connector 18 of the casing 13 and has a collection volume equal to or larger than the initial volume of the liquid flow to be sampled, e.g. less than the first 12 mL of first-void urine, e.g. less than or equal to 4 mL of first-void urine, e.g. less than or equal to 3 mL of first-void urine, e.g. between 2.6 mL and 2.9 mL of first-void urine.

A dissolvable material layer of preservation agents or a buffer solution may be applied to a bottom of the receptacle 19 prior to the sampling of the initial volume. This allows preserving the sampled initial volume during the time required for sending the closed receptacle to the laboratory charged with the urinalysis. Suitable materials for the components of the sampling device comprise, without being limited thereto, polypropylene, biodegradable polymers, or form-shaped bagasse, and withstand urine at body temperatures, e.g. at least 40 degree Celsius. Plastic components of the sampling device may be manufactured at large scale by injection molding. Preferably, the materials for the components of the sampling device are also easily compressed or folded such that they can be sent, as a kit or as separate parts, to the user by mail (e.g. when less than 28 mm thick), who then expands or unfolds the components for assembly.

In embodiments of the invention, in which the inlet conduit 12 is not formed as a funnel 62, such an inlet conduit may be adapted to receive and secure a separate funnel at the distal opening, e.g. a funnel support may comprise a V-groove for placing and aligning a funnel and a clip for securing and maintaining an open configuration of the funnel. An accessory funnel may be part of the kit 60 and deliverable in compact form, e.g. folded or collapsed before being unfolded or expanded prior to use. A separate funnel has the advantage that it may be provided as a disposable funnel made from biodegradable polymers and suitable for direct flushing, in addition of being fitted to the anatomy of a specific gender, e.g. male or female. It is noticed that the closure member 17 as one of the components of the kit 60 is removably insertable into the valve casing 13 at an opening at its top. In this case, the previously mentioned resilient protrusion 13d (e.g. stopper element) is flexed under the pressure of the cap 16 when the closure member 17 is inserted into the guiding structure of the casing. Once the cap 16 has passed the resilient protrusion 13d it snaps back to its original position, thus restricting the axial movement of the inserted closure member 17 to the interior of the casing 13 and preventing the closure member from accidentally falling out of the casing. To facilitate insertion during assembly, the resilient protrusion 13d may have a downwards sloping upper side.

In the above-described embodiments, the use of very narrow small-volume collection tubes is supported. The liquid sampling device is designed for increased collection speed such that also very small initial volumes can be captured accurately in correspondingly small collection tubes, e.g. in commercially available tubes such as Becton Dickinson 3 mL tubes, or even smaller tubes, e.g. in the 1.0 mL-1.5 mL volume range. These small and narrow tubes are advantageous as they can be used directly in liquid-handling robots for automated sampling, e.g. in high-throughput analyzer instruments.

While the invention has been illustrated and described in detail in the drawings and foregoing description, such illustration and description are to be considered illustrative or exemplary and not restrictive. The foregoing description details certain embodiments of the invention. It will be appreciated, however, that no matter how detailed the foregoing appears in text, the invention may be practiced in many ways. Other variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. The scope of the invention is defined by the appended claims solely.

## Claims

1. A device (10) for sampling an initial volume of a liquid flow, comprising:
a casing (13) comprising a fluid inlet (13a) for receiving the liquid flow, a sample outlet (13c) for draining the initial volume of the liquid flow, a fluid outlet (13b) for draining a subsequent volume of the liquid flow, and a passageway (22) for providing fluid communication between the fluid inlet (13a) and the fluid outlet (13b) and between the fluid inlet (13a) and the sample outlet (13c),
an elongated closure member (17) slidably supported in the casing (13), the closure member (17) comprising a head portion (14) and a stem (15), and
a lifting member connected to or formed in the stem (15),
wherein the head portion (14) is configured to obstruct the liquid flow through the fluid outlet (13b) when the closure member (17) is slid into a sampling position, thus preventing the initial volume of the liquid flow from being transferred to the fluid outlet (13b),
wherein a lower portion (15b) of the stem (15), distal to the head portion (14), is configured to obstruct the liquid flow through the sample outlet (13c) when the closure member (17) is slid into a diverting position, thus ensuring that the subsequent volume of the liquid flow is transferred to the fluid outlet (13b),
wherein said lifting member is a float adapted for moving the closure member (17) from the sampling position to the diverting position while the initial volume of the liquid flow is being collected into a receptacle (19), connectable to the sample outlet (13c) of the casing (13),
**characterized in that** an upper portion (15a) of the stem (15), proximate to the head portion (14), comprises at least one longitudinal slit (20), said at least one slit (20) stretching across the sample outlet (13c) to fluidly connect a front side of the stem (15) facing the fluid inlet (13a) in the casing (13) to an opposing rear side of the stem (15) when the closure member (17) is slid into the sampling position, thus allowing the initial volume of the liquid flow to exit said sample outlet (13c) via said at least one slit (20).

2. The device (10) according to claim 1, wherein the head portion (14) is positioned in the passageway (22) when the closure member (17) is slid to the sampling position, and wherein the lower portion (15b) of the stem (15) is extending through and at least partially into said passageway (22) when the closure member (17) is slid into the diverting position.

3. The device (10) according to claim 1, wherein the lifting member is an air cavity (15d) formed in the lower portion (15b) of the stem (15).

4. The device (10) according to claim 1, wherein the head portion (14) comprises a slanted barrier wall (14a, 14b) having edges that are lying flush with corresponding inner surfaces of the passageway (22) inside the casing (13), when the closure member (17) is slid into the sampling position;
optionally, wherein:
the slanted barrier wall (14a, 14b) comprises a first flat wall segment (14a) proximate to the fluid inlet (13a) and a second flat wall segment (14b) distal to the fluid inlet (13a), an inclination of the second flat wall segment (14b) with respect to a longitudinal axis of the closure member (17) being steeper than an inclination of the first flat wall segment (14a); or
the upper portion (15a) of the stem (15) abuts on a front side of the barrier wall (14a, 14b), which front side is facing the fluid inlet (13a) and delimiting the at least one slit (20) at the top.

5. The device (10) according to claim 1, wherein:
lateral side surfaces of the at least one slit (20) are plane surfaces perpendicular to a longitudinal axis of the closure member (17); optionally, wherein the upper portion (15a) of the stem (15) comprises at least two longitudinal slits (20a, 20b); or
the at least one slit (20) is delimited by a flat surface (15c) at the bottom.

6. The device (10) according to claim 1, wherein:
an outer surface of the stem (15) of the closure member (17) is lying flush with an inner surface of the sample outlet (13c) when the closure member (17) is slid into the diverting position; or
the casing (13) comprises a resilient protrusion (13d) on an inner surface of said casing (13) for limiting a farther movement of the closure member (17) slid into the diverting position.

7. The device (10) according to claim 1, wherein the lower portion (15b) of said stem (15) is directly and continuously connected to the upper portion (15a) of said stem (15); optionally, wherein the stem (15) has a cylindrical shape.

8. The device (10) according to claim 1, wherein the sample outlet (13c) is formed as a through-hole in a base (13f) of the casing (13) and the casing (13) further comprises a connector (18) projecting outwardly from said base (13f), said connector (18) being adapted to engage a receptacle (19) for collecting the initial volume of the liquid flow; optionally, wherein the connector (18) has an internal thread for threadedly engaging said receptacle (19).

9. The device (10) according to claim 1, wherein the head portion (14) of the closure member (17) is terminated by a cap (16) whose outer edge is lying flush with an inner surface of the casing (13), whereby the closure member (17) is confined to slide along a longitudinal axis of the closure member (17); optionally, wherein the outer edge of the cap (16) is non-circular.

10. The device (10) according to claim 1, further comprising an inlet conduit (12) connected to the fluid inlet (13a) and an outlet conduit (11) connected to the fluid outlet (13b);
optionally, wherein:
the casing (13), the inlet conduit (12), and the outlet conduit (11) are formed monolithically as a single piece; or
the inlet conduit (12) comprises a funnel.

11. A kit of parts (60) for assembling a device (10) for sampling an initial volume of a liquid flow, comprising:
a casing (13) comprising a fluid inlet (13a) for receiving the liquid flow, a sample outlet (13c) for draining the initial volume of the liquid flow, a fluid outlet (13b) for draining a subsequent volume of the liquid flow, and a passageway (22) for providing fluid communication between the fluid inlet (13a) and the fluid outlet (13b) and between the fluid inlet (13a) and the sample outlet (13c),
an inlet conduit (12) connectable or connected to the fluid inlet (13a),
an outlet conduit (11) connectable or connected to the fluid outlet (13b),
an elongated closure member (17) slidably receivable by the casing (13), said closure member (17) comprising a head portion (14) and a stem (15), and
a lifting member connectable to or formed in the stem (15),
wherein the head portion (14) is adapted for obstructing the liquid flow through the fluid outlet (13b) when the closure member (17) is slid into a sampling position,
wherein a lower portion (15b) of the stem (15), distal to the head portion (14), is adapted for obstructing the liquid flow through the sample outlet (13c) without obstructing the liquid flow through the fluid outlet (13b) when the closure member (17) is slid into a diverting position,
wherein said lifting member is a float adapted for moving the closure member (17) from the sampling position to the diverting position while the initial volume of the liquid flow is being collected into a receptacle (19), connectable to the sample outlet (13c) of the casing (13),
**characterized in that** an upper portion (15a) of the stem (15), proximate to the head portion (14), comprises at least one longitudinal slit (20), said at least one slit (20) stretching across the sample outlet (13c) to fluidly connect a front side of the stem (15) facing the fluid inlet (13a) in the casing (13) to an opposing rear side of the stem (15) when the closure member (17) is slid into the sampling position, thus allowing an initial volume of the liquid flow to exit said sample outlet (13c) via said at least one slit (20).

12. The kit (60) according to claim 11, wherein the inlet conduit (12), the outlet conduit (11), and the casing (13) are provided as a single, monolithically formed piece (61).

13. The kit (60) according to claim 11, wherein the lifting member and the closure member (17) are provided as a single, monolithically formed piece; optionally, wherein the lifting member comprises an air cavity (15d) formed in the lower portion (15b) of the stem (15).

14. The kit (60) according to claim 11, wherein the casing (13) comprises a guide channel (13e) for guiding the head portion (14), the guide channel (13e) having an inner surface shape fitted to outer edges of the head portion (14) to support a sliding movement of the closure member (17) in the casing (13); optionally, wherein the casing (13) comprises a resilient protrusion (13d) on an inner surface of said casing (13) for limiting a farther movement of the closure member (17) slid into the diverting position.

15. The kit (60) according to claim 11, further comprising a receptacle (19) with a volume ranging between 1 mL and 50 mL, the receptacle (19) being connectable to a sampling connector (18) that is projecting outwardly from a base (13f) of the casing (13);
optionally, wherein:
an outer surface of the receptacle (19) comprises a threaded portion for engaging a corresponding internal thread of the sampling connector (18); or
the receptacle (19) is a collection tube and/or wherein the receptacle (19) contains a preservation liquid or nucleic acid (e.g. DNA) stabilization agent.

16. The kit (60) according to claim 11, wherein:
the kit (60) further comprises a funnel connectable to the inlet conduit (12), the inlet conduit (12) being adapted for receiving and securing the funnel;
the inlet conduit (12) has a flexible shape; or
one or more parts of the kit (60) are made of a biodegradable polymer material.

## Patentansprüche

1. Vorrichtung (10) zur Probenentnahme eines Ausgangsvolumens eines Flüssigkeitsstroms, umfassend:
ein Gehäuse (13) mit einem Fluideinlass (13a) zur Aufnahme des Flüssigkeitsstroms, einem Probenauslass (13c) zum Ableiten des Ausgangsvolumens des Flüssigkeitsstroms, einem Fluidauslass (13b) zum Ableiten eines nachfolgenden Volumens des Flüssigkeitsstroms sowie einem Durchgang (22) zur Bereitstellen einer Fluidverbindung zwischen dem Fluideinlass (13a) und dem Fluidauslass (13b) sowie zwischen dem Fluideinlass (13a) und dem Probenauslass (13c),
ein längliches Verschlusselement (17), das verschiebbar im Gehäuse (13) gelagert ist, wobei das Verschlusselement (17) einen Kopfabschnitt (14) und einen Schaft (15) umfasst, und
ein mit dem Schaft (15) verbundenes oder in diesem ausgebildetes Hebeelement,
wobei der Kopfabschnitt (14) so ausgebildet ist, dass er den Flüssigkeitsstrom durch den Fluidauslass (13b) behindert, wenn das Verschlusselement (17) in eine Probenentnahmeposition verschoben wird, wodurch verhindert wird, dass das Ausgangsvolumen des Flüssigkeitsstroms zum Fluidauslass (13b) geleitet wird;
wobei ein unterer Abschnitt (15b) des Schafts (15), der vom Kopfabschnitt (14) entfernt liegt, so ausgebildet ist, dass er den Flüssigkeitsstrom durch den Probenauslass (13c) behindert, wenn das Verschlusselement (17) in eine Umleitungsposition geschoben wird, wodurch sichergestellt wird, dass das nachfolgende Volumen des Flüssigkeitsstroms zum Fluidauslass (13b) geleitet wird,
wobei das Hebeelement ein Schwimmer ist, der dazu ausgelegt ist, das Verschlusselement (17) von der Probenentnahmeposition in die Umleitungsposition zu bewegen, während das Ausgangsvolumen des Flüssigkeitsstroms in einem Behälter (19) aufgefangen wird, der mit dem Probenauslass (13c) des Gehäuses (13) verbunden werden kann,
**dadurch gekennzeichnet, dass** ein oberer Abschnitt (15a) des Schafts (15), der dem Kopfabschnitt (14) nahe liegt, mindestens einen Längsschlitz (20) umfasst, wobei sich der mindestens eine Schlitz (20) über den Probenauslass (13c) erstreckt, um eine dem Fluideinlass (13a) im Gehäuse (13) zugewandte Vorderseite des Schafts (15) mit einer gegenüberliegenden Rückseite des Schafts (15) fluidisch zu verbinden, wenn das Verschlusselement (17) in die Probenentnahmeposition geschoben wird, wodurch das Ausgangsvolumen des Flüssigkeitsstroms aus den Probenauslass (13c) über den mindestens einen Schlitz (20) austreten kann.

2. Vorrichtung (10) nach Anspruch 1, wobei sich der Kopfabschnitt (14) im Durchgang (22) befindet, wenn das Verschlusselement (17) in die Probenentnahmeposition geschoben wird, und wobei sich der untere Abschnitt (15b) des Schafts (15) durch den Durchgang (22) erstreckt und zumindest teilweise in diesen hineinragt, wenn das Verschlusselement (17) in die Umleitungsposition geschoben wird.

3. Vorrichtung (10) nach Anspruch 1, wobei das Hebeelement ein Luftraum (15d) ist, der im unteren Abschnitt (15b) des Schafts (15) ausgebildet ist.

4. Vorrichtung (10) nach Anspruch 1, wobei der Kopfabschnitt (14) eine schräge Barrierewand (14a, 14b) mit Kanten umfasst, die bündig mit den entsprechenden Innenflächen des Durchgangs (22) im Inneren des Gehäuses (13) liegen, wenn das Verschlusselement (17) in die Probeentnahmeposition geschoben wird;
optional, wobei:
die schräge Barrierewand (14a, 14b) ein erstes flaches Wandsegment (14a) nahe dem Fluideinlass (13a) und ein zweites flaches Wandsegment (14b) entfernt vom Fluideinlass (13a) umfasst, wobei eine Neigung des zweiten flachen Wandsegments (14b) in Bezug auf eine Längsachse des Verschlusselements (17) steiler ist als eine Neigung des ersten flachen Wandsegments (14a); oder
der obere Abschnitt (15a) des Schafts (15) an einer Vorderseite der Barrierewand (14a, 14b) anliegt, wobei diese Vorderseite dem Fluideinlass (13a) zugewandt ist und den mindestens einen Schlitz (20) an der Oberseite begrenzt.

5. Vorrichtung (10) nach Anspruch 1, wobei:
seitliche Flächen des mindestens einen Schlitzes (20) ebene Flächen sind, die senkrecht zu einer Längsachse des Verschlusselements (17) stehen; optional, wobei der obere Abschnitt (15a) des Schafts (15) mindestens zwei Längsschlitze (20a, 20b) umfasst; oder der mindestens eine Schlitz (20) unten durch eine flache Fläche (15c) begrenzt wird.

6. Vorrichtung (10) nach Anspruch 1, wobei:
eine Außenfläche des Schafts (15) des Verschlusselements (17) bündig mit einer Innenfläche des Probenauslasses (13c) abschließt, wenn das Verschlusselement (17) in die Umleitungsposition geschoben ist;
oder
das Gehäuse (13) einen elastischen Vorsprung (13d) an einer Innenfläche des Gehäuses (13) umfasst, um eine weitere Bewegung des in die Umleitungsposition verschobenen Verschlusselements (17) zu begrenzen.

7. Vorrichtung (10) nach Anspruch 1, wobei der untere Abschnitt (15b) des Schafts (15) direkt und durchgehend mit dem oberen Abschnitt (15a) des Schafts (15) verbunden ist; optional wobei der Schaft (15) eine zylindrische Form aufweist.

8. Vorrichtung (10) nach Anspruch 1, wobei der Probenauslass (13c) als Durchgangsbohrung in einer Basis (13f) des Gehäuses (13) ausgebildet ist und das Gehäuse (13) ferner einen Anschluss (18), der aus der genannten Basis (13f) nach außen ragt, wobei der Anschluss (18) dazu ausgelegt ist, in einem Behälter (19) zum Auffangen des Ausgangsvolumens des Flüssigkeitsstroms einzugreifen; wobei der Anschluss (18) optional ein Innengewinde zum schraubbaren Eingriff mit dem Behälter (19) aufweist.

9. Vorrichtung (10) nach Anspruch 1, wobei der Kopfabschnitt (14) des Verschlusselements (17) durch eine Kappe (16) abgeschlossen ist, deren Außenkante bündig mit einer Innenfläche des Gehäuses (13) abschließt, wodurch das Verschlusselement (17) so begrenzt ist, dass es entlang einer Längsachse des Verschlusselements (17) gleiten kann; optional, wobei die Außenkante der Kappe (16) nicht kreisförmig ist.

10. Vorrichtung (10) nach Anspruch 1, ferner umfassend eine Einlassleitung (12), die mit dem Fluideinlass (13a) verbunden ist, und eine Auslassleitung (11), die mit dem Fluidauslass (13b) verbunden ist;
optional, wobei:
das Gehäuse (13), die Einlassleitung (12) und die Auslassleitung (11)monolithisch als ein einziges Teil ausgebildet sind; oder
die Einlassleitung (12) einen Trichter umfasst.

11. Kit aus Teilen (60) zum Zusammenbau einer Vorrichtung (10) zur Probenentnahme eines Ausgangsvolumens eines Flüssigkeitsstroms, umfassend:
ein Gehäuse (13) mit einem Fluideinlass (13a) zur Aufnahme des Flüssigkeitsstroms, einem Probenauslass (13c) zum Ableiten des Ausgangsvolumens des Flüssigkeitsstroms, einen Fluidauslass (13b) zum Ableiten eines nachfolgenden Volumens des Flüssigkeitsstroms sowie einen Durchgang (22) zum Bereitstellen einer Fluidverbindung zwischen dem Fluideinlass (13a) und dem Fluidauslass (13b) sowie zwischen dem Fluideinlass (13a) und dem Probenauslass (13c),
eine Einlassleitung (12), die mit dem Fluideinlass (13a) verbindbar oder verbunden ist,
eine Auslassleitung (11), die mit dem Fluidauslass (13b) verbindbar oder verbunden ist,
ein längliches Verschlusselement (17), das vom Gehäuse (13) verschiebbar aufgenommen werden kann, wobei das Verschlusselement (17) einen Kopfabschnitt (14) und einen Schaft (15) umfasst, und
ein mit dem Schaft (15) verbindbares oder in diesem ausgebildetes Hebeelement,
wobei der Kopfabschnitt (14) dazu ausgelegt ist, den Flüssigkeitsstrom durch den Fluidauslass (13b) zu behindern, wenn das Verschlusselement (17) in eine Probenentnahmeposition geschoben wird,
wobei ein unterer Abschnitt (15b) des Schafts (15), der vom Kopfabschnitt (14) entfernt liegt, dazu ausgelegt ist, den Flüssigkeitsstrom durch den Probenauslass (13c) zu behindern, ohne den Flüssigkeitsstrom durch den Fluidauslass (13b) zu behindern, wenn das Verschlusselement (17) in eine Umleitungsposition geschoben wird,
wobei das Hebeelement ein Schwimmer ist, der dazu ausgelegt ist, das Verschlusselement (17) aus der Probenentnahmeposition in die Umleitungsposition zu bewegen, während das Ausgangsvolumen des Flüssigkeitsstroms in einem Behälter (19) gesammelt wird, der mit dem Probenauslass (23c) des Gehäuses (13) verbunden werden kann,
**dadurch gekennzeichnet, dass** ein oberer Abschnitt (15a) des Schafts (15) in der Nähe des Kopfabschnitts (24) mindestens einen Längsschlitz (20) umfasst, wobei sich der mindestens eine Schlitz (20) über den Probenauslass (13c) erstreckt, um eine dem Fluideinlass (13a) im Gehäuse (13) zugewandte Vorderseite des Schafts (15) mit einer gegenüberliegenden Rückseite des Schafts (15) fluidisch zu verbinden, wenn das Verschlusselement (17) in die Probenentnahmeposition geschoben wird, wodurch ein Ausgangsvolumen des Flüssigkeitsstroms über den mindestens einen Schlitz (20) aus dem Probenauslass (13c) austreten kann.

12. Kit (60) nach Anspruch 11, wobei die Einlassleitung (12), die Auslassleitung (11) und das Gehäuse (13) als ein einziges, monolithisch geformtes Teil (61) ausgebildet sind.

13. Kit (60) nach Anspruch 11, wobei das Hebeelement und das Verschlusselement (17) als ein einziges, monolithisch geformtes Teil ausgebildet sind; wobei das Hebeelement optional einen im unteren Abschnitt (15b) des Schafts (15) ausgebildeten Luftraum (15d) umfasst.

14. Kit (60) nach Anspruch 11, wobei das Gehäuse (13) einen Führungskanal (13e) zur Führung des Kopfabschnitts (14) umfasst, wobei der Führungskanal (13e) eine Innenflächenform aufweist, die an die Außenkanten des Kopfabschnitts (14) angepasst ist, um eine Gleitbewegung des Verschlusselements (17) in dem Gehäuse (13) zu unterstützen; optional, wobei das Gehäuse (13) einen elastischen Vorsprung (13d) an einer Innenfläche des Gehäuses (13) umfasst, um eine weitere Bewegung des Verschlusselements (17) zu begrenzen, das in die Umleitungsposition verschoben wird.

15. Kit (60) nach Anspruch 11, das ferner einen Behälter (19) mit einem Volumen zwischen 1 ml und 50 ml umfasst, wobei der Behälter (19) mit einem Probenentnahmeanschluss (18) verbindbar ist, der von einer Basis (13f) des Gehäuses (13) nach außen vorsteht; wobei optional:
eine Außenfläche des Behälters (19) einen Gewindeabschnitt zum Eingriff mit einem entsprechenden Innengewinde des Probenentnahmeanschlusses (18) aufweist; oder
der Behälter (19) ein Sammelröhrchen ist und/oder wobei der Behälter (19) eine Konservierungsflüssigkeit oder ein Stabilisierungsmittel für Nukleinsäuren (z. B. DNA) enthält.

16. Kit (60) nach Anspruch 11, wobei:
das Kit (60) ferner einen Trichter umfasst, der mit der Einlassleitung (12) verbindbar ist, wobei die Einlassleitung (12) zur Aufnahme und Befestigung des Trichters ausgelegt ist; die Einlassleitung (12) eine flexible Form aufweist; oder
ein oder mehrere Teile des Kits (60) aus einem biologisch abbaubaren Polymermaterial bestehen.

## Revendications

1. Dispositif (10) destiné à échantillonner un volume initial d'un écoulement de liquide, comprenant :
un boîtier (13) comprenant une entrée de fluide (13a) destinée à recevoir l'écoulement de liquide, une sortie d'échantillon (13c) destinée à évacuer le volume initial de l'écoulement de liquide, une sortie de fluide (13b) destinée à évacuer un volume ultérieur de l'écoulement de liquide, et un passage (22) destiné à assurer une communication fluidique entre l'entrée de fluide (13a) et la sortie de fluide (13b) et entre l'entrée de fluide (13a) et la sortie d'échantillon (13c),
un organe de fermeture allongé (17) supporté de manière coulissante dans le boîtier (13), l'organe de fermeture (17) comprenant une partie de tête (14) et une tige (15), et
un organe de levage relié à la tige (15) ou formé dans celle-ci,
dans lequel la partie de tête (14) est configurée pour obstruer l'écoulement de liquide à travers la sortie de fluide (13b) lorsque l'organe de fermeture (17) est coulissé dans une position d'échantillonnage, empêchant ainsi le volume initial de l'écoulement de liquide d'être transféré vers la sortie de fluide (13b),
dans lequel une partie inférieure (15b) de la tige (15), distale par rapport à la partie de tête (14), est configurée pour obstruer l'écoulement de liquide à travers la sortie d'échantillon (13c) lorsque l'organe de fermeture (17) est coulissé dans une position de dérivation, garantissant ainsi que le volume ultérieur de l'écoulement de liquide est transféré vers la sortie de fluide (13b),
dans lequel ledit organe de levage est un flotteur adapté pour déplacer l'organe de fermeture (17) de la position d'échantillonnage à la position de dérivation pendant que le volume initial de l'écoulement de liquide est recueilli dans un récipient (19), pouvant être relié à la sortie d'échantillon (13c) du boîtier (13),
**caractérisé en ce qu'**une partie supérieure (15a) de la tige (15), proximale par rapport à la partie de tête (14), comprend au moins une fente longitudinale (20), ladite au moins une fente (20) s'étendant en travers de la sortie d'échantillon (13c) pour relier fluidiquement un côté avant de la tige (15) faisant face à l'entrée de fluide (13a) dans le boîtier (13) à un côté arrière opposé de la tige (15) lorsque l'organe de fermeture (17) est coulissé dans la position d'échantillonnage, permettant ainsi au volume initial de l'écoulement de liquide de sortir par ladite sortie d'échantillon (13c) via ladite au moins une fente (20).

2. Dispositif (10) selon la revendication 1, dans lequel la partie de tête (14) est positionnée dans le passage (22) lorsque l'organe de fermeture (17) est coulissé dans la position d'échantillonnage, et dans lequel la partie inférieure (15b) de la tige (15) s'étend à travers ledit passage (22) et au moins partiellement dans celui-ci lorsque l'organe de fermeture (17) est coulissé dans la position de dérivation.

3. Dispositif (10) selon la revendication 1, dans lequel l'organe de levage est une cavité d'air (15d) formée dans la partie inférieure (15b) de la tige (15).

4. Dispositif (10) selon la revendication 1, dans lequel la partie de tête (14) comprend une paroi de barrière inclinée (14a, 14b) ayant des bords qui affleurent des surfaces intérieures correspondantes du passage (22) à l'intérieur du boîtier (13), lorsque l'organe de fermeture (17) est coulissé dans la position d'échantillonnage ;
éventuellement, dans lequel :
la paroi de barrière inclinée (14a, 14b) comprend un premier segment de paroi plat (14a) proximal par rapport à l'entrée de fluide (13a) et un deuxième segment de paroi plat (14b) distal par rapport à l'entrée de fluide (13a), une inclinaison du deuxième segment de paroi plat (14b) par rapport à un axe longitudinal de l'organe de fermeture (17) étant plus forte qu'une inclinaison du premier segment de paroi plat (14a) ; ou
la partie supérieure (15a) de la tige (15) vient en butée contre un côté avant de la paroi de barrière (14a, 14b), lequel côté avant fait face à l'entrée de fluide (13a) et délimite l'au moins une fente (20) en haut.

5. Dispositif (10) selon la revendication 1, dans lequel :
des surfaces latérales de l'au moins une fente (20) sont des surfaces planes perpendiculaires à un axe longitudinal de l'organe de fermeture (17) ; éventuellement, dans lequel la partie supérieure (15a) de la tige (15) comprend au moins deux fentes longitudinales (20a, 20b) ; ou
l'au moins une fente (20) est délimitée par une surface plane (15c) en bas.

6. Dispositif (10) selon la revendication 1, dans lequel :
une surface extérieure de la tige (15) de l'organe de fermeture (17) affleure une surface intérieure de la sortie d'échantillon (13c) lorsque l'organe de fermeture (17) est coulissé dans la position de dérivation ; ou
le boîtier (13) comprend une saillie élastique (13d) sur une surface intérieure dudit boîtier (13) pour limiter un déplacement supplémentaire de l'organe de fermeture (17) coulissé dans la position de dérivation.

7. Dispositif (10) selon la revendication 1, dans lequel la partie inférieure (15b) de ladite tige (15) est reliée directement et de manière continue à la partie supérieure (15a) de ladite tige (15) ; éventuellement, dans lequel la tige (15) présente une forme cylindrique.

8. Dispositif (10) selon la revendication 1, dans lequel la sortie d'échantillon (13c) est formée sous la forme d'un trou traversant dans une base (13f) du boîtier (13) et le boîtier (13) comprend en outre un raccord (18) faisant saillie vers l'extérieur à partir de ladite base (13f), ledit raccord (18) étant adapté pour venir en prise avec un récipient (19) destiné à recueillir le volume initial de l'écoulement de liquide ; éventuellement, dans lequel le raccord (18) présente un filetage intérieur destiné à venir en prise par vissage avec ledit récipient (19).

9. Dispositif (10) selon la revendication 1, dans lequel la partie de tête (14) de l'organe de fermeture (17) se termine par un capuchon (16) dont le bord extérieur affleure une surface intérieure du boîtier (13), de sorte que l'organe de fermeture (17) est contraint de coulisser le long d'un axe longitudinal de l'organe de fermeture (17) ; éventuellement, dans lequel le bord extérieur du capuchon (16) est non circulaire.

10. Dispositif (10) selon la revendication 1, comprenant en outre un conduit d'entrée (12) relié à l'entrée de fluide (13a) et un conduit de sortie (11) relié à la sortie de fluide (13b) ;
éventuellement, dans lequel :
le boîtier (13), le conduit d'entrée (12) et le conduit de sortie (11) sont formés de manière monolithique en une seule pièce ; ou
le conduit d'entrée (12) comprend un entonnoir.

11. Kit de pièces (60) destiné à l'assemblage d'un dispositif (10) destiné à échantillonner un volume initial d'un écoulement de liquide, comprenant :
un boîtier (13) comprenant une entrée de fluide (13a) destinée à recevoir l'écoulement de liquide, une sortie d'échantillon (13c) destinée à évacuer le volume initial de l'écoulement de liquide, une sortie de fluide (13b) destinée à évacuer un volume ultérieur de l'écoulement de liquide, et un passage (22) destiné à assurer une communication fluidique entre l'entrée de fluide (13a) et la sortie de fluide (13b) et entre l'entrée de fluide (13a) et la sortie d'échantillon (13c),
un conduit d'entrée (12) pouvant être relié ou étant relié à l'entrée de fluide (13a),
un conduit de sortie (11) pouvant être relié ou étant relié à la sortie de fluide (13b),
un organe de fermeture allongé (17) pouvant être reçu de manière coulissante par le boîtier (13), ledit organe de fermeture (17) comprenant une partie de tête (14) et une tige (15), et
un organe de levage pouvant être relié à la tige (15) ou formé dans celle-ci,
dans lequel la partie de tête (14) est adaptée pour obstruer l'écoulement de liquide à travers la sortie de fluide (13b) lorsque l'organe de fermeture (17) est coulissé dans une position d'échantillonnage,
dans lequel une partie inférieure (15b) de la tige (15), distale par rapport à la partie de tête (14), est adaptée pour obstruer l'écoulement de liquide à travers la sortie d'échantillon (13c) sans obstruer l'écoulement de liquide à travers la sortie de fluide (13b) lorsque l'organe de fermeture (17) est coulissé dans une position de dérivation,
dans lequel ledit organe de levage est un flotteur adapté pour déplacer l'organe de fermeture (17) de la position d'échantillonnage à la position de dérivation pendant que le volume initial de l'écoulement de liquide est recueilli dans un récipient (19), pouvant être relié à la sortie d'échantillon (13c) du boîtier (13),
**caractérisé en ce qu'**une partie supérieure (15a) de la tige (15), proximale par rapport à la partie de tête (14), comprend au moins une fente longitudinale (20), ladite au moins une fente (20) s'étendant en travers de la sortie d'échantillon (13c) pour relier fluidiquement un côté avant de la tige (15) faisant face à l'entrée de fluide (13a) dans le boîtier (13) à un côté arrière opposé de la tige (15) lorsque l'organe de fermeture (17) est coulissé dans la position d'échantillonnage, permettant ainsi à un volume initial de l'écoulement de liquide de sortir par ladite sortie d'échantillon (13c) via ladite au moins une fente (20).

12. Kit (60) selon la revendication 11, dans lequel le conduit d'entrée (12), le conduit de sortie (11) et le boîtier (13) sont fournis sous la forme d'une seule pièce (61) formée de manière monolithique.

13. Kit (60) selon la revendication 11, dans lequel l'organe de levage et l'organe de fermeture (17) sont fournis sous la forme d'une seule pièce formée de manière monolithique ; éventuellement, dans lequel l'organe de levage comprend une cavité d'air (15d) formée dans la partie inférieure (15b) de la tige (15).

14. Kit (60) selon la revendication 11, dans lequel le boîtier (13) comprend un canal de guidage (13e) destiné à guider la partie de tête (14), le canal de guidage (13e) présentant une forme de surface intérieure ajustée aux bords extérieurs de la partie de tête (14) afin de supporter un mouvement coulissant de l'organe de fermeture (17) dans le boîtier (13) ; éventuellement, dans lequel le boîtier (13) comprend une saillie élastique (13d) sur une surface intérieure dudit boîtier (13) pour limiter un déplacement supplémentaire de l'organe de fermeture (17) coulissé dans la position de dérivation.

15. Kit (60) selon la revendication 11, comprenant en outre un récipient (19) ayant un volume compris entre 1 ml et 50 ml, le récipient (19) pouvant être relié à un raccord d'échantillonnage (18) faisant saillie vers l'extérieur à partir d'une base (13f) du boîtier (13) ;
éventuellement, dans lequel :
une surface extérieure du récipient (19) comprend une partie filetée destinée à venir en prise avec un filetage intérieur correspondant du raccord d'échantillonnage (18) ; ou
le récipient (19) est un tube de collecte et/ou dans lequel le récipient (19) contient un liquide de conservation ou un agent de stabilisation d'acide nucléique (par exemple, d'ADN).

16. Kit (60) selon la revendication 11, dans lequel :
le kit (60) comprend en outre un entonnoir pouvant être relié au conduit d'entrée (12), le conduit d'entrée (12) étant adapté pour recevoir et fixer l'entonnoir ;
le conduit d'entrée (12) présente une forme flexible ; ou
une ou plusieurs pièces du kit (60) sont constituées d'un matériau polymère biodégradable.
